(19) Europäisches Patentamt
European Patent Office
Office européen des brevets

(11) Publication number: **0 413 583 A2**

(12) **EUROPEAN PATENT APPLICATION**

(21) Application number: **90308997.7**

(22) Date of filing: **16.08.90**

(51) Int. Cl.[5]: **A61K 31/35**, A61K 47/12, A61K 9/06, A61K 9/72

(30) Priority: **18.08.89 US 395937**
**22.12.89 US 454771**

(43) Date of publication of application:
**20.02.91 Bulletin 91/08**

(84) Designated Contracting States:
**AT BE CH DE DK ES FR GB GR IT LI LU NL SE**

(71) Applicant: **DEY LABORATORIES; INC.**
**2751 Napa Valley Corporate Drive**
**Napa, California 94558(US)**

Applicant: **Zeleznick, Lowell**
**26 Lakeview**
**Irvine, California 92714(US)**

(72) Inventor: **Raff, Allan M.**
**1451 Creekside Drive**
**Walnut Creek, California 94556(US)**
Inventor: **Zeleznick, Lowell**
**26 Lakeview**
**Irvine, California 92714(US)**

(74) Representative: **Allard, Susan Joyce et al**
**BOULT, WADE & TENNANT, 27 Furnival Street**
**London EC4A 1PQ(GB)**

# (54) Clear, stable cromolyn formulation.

(57) A substantially clear, sterile, aqueous tonicity adjusted composition of 1,3-bis(2-carboxychromon-5-yloxy)-propan-2-ol, other bis-cromolyn derivatives, or pharmaceutically acceptable salts thereof, is provided which contains over 20 ppm transition metal ions or ions of Group IIA, IB, IIB or IV B. The composition is useful as an anti-inflammatory agent in the treatment of bronchial asthmatics and allergic or immune reactions in the eye and nose.

EP 0 413 583 A2

Xerox Copy Centre

## CLEAR, STABLE CROMOLYN FORMULATION

This invention relates to novel stable aqueous pharmaceutical formulations, and particularly to clear, aqueous, stable solutions of cromolyn.

The active ingredient known as cromolyn or chromolyn, 1,3-bis(2-carboxychromon-5-yloxy)propan-2-ol (or any of its pharmaceutically acceptable salts), is useful as an anti-inflammatory agent for treatment of inflammation of the eyes, nose, or in the management of patients with bronchial asthma, which conditions may result from allergy or immune reactions to various substances. One of the problems with cromolyn in an aqueous solution, however, is that the solution can become rapidly cloudy, and therefore pharmaceutically unacceptable. Furthermore, the polar nature of the compound and its relatively high molecular weight indicates that it could combine with known cationic preservatives for aqueous pharmaceutical solutions to form insoluble precipitates, thus further aggravating the problem of the cloudiness.

In U.S. Patent No. 3,975,536 a clear aqueous formulation of cromolyn is disclosed; however, the solution is restricted in that it must contain less than 0.40 ppm ions of metals of groups IIA, IB, IIB and IVB of the Periodic Table and of transition metals in order to maintain the clarity of the solution. In that patent an alternative method of making a clear solution of cromolyn is provided using a chelating agent; however, even in the presence of the chelating agent the solution must contain less than 20 ppm of ions of metals of groups IIA, IB, IIB and IVB of the Periodic Table and of the transition metals in order to maintain clarity. However, the task of maintaining less than 20 ppm of those types of metal ions in solution, and especially, of maintaining less than 0.40 ppm of those ions in solution, requires careful control not only of the preparative methods, but also of the methods of storage and use of the solution. Furthermore, particularly in the treatment of the nose, eye and for oral inhalation, it would be useful if the solution containing the active cromolyn ingredient would be isotonic (i.e., having an osmotic pressure of about 300 mOsm) or only slightly hypotonic (200-300 mOsm in the case of an ophthalmic solution) to minimize irritation. See Am Rev. Respiratory Dis (1988) 137 1309-1311. The two above-described compositions containing, respectively, less than 20 ppm and less than 0.40 ppm of certain metal ions disclosed in Patent 3,975,536 are not isotonic.

It would thus be advantageous to provide aqueous formulations of cromolyn and related bis-cromolyn compounds, or their pharmaceutically acceptable salts which are clear, stable, and tonicity adjusted, while still capable of containing Group IIA, IB, IIB or IV B metal ions, or transition metal ions at concentrations greater than about 20 ppm.

It would also be advantageous to provide a stable aqueous isotonic formulation of cromolyn which is relatively low in sodium ion content.

Accordingly, the present invention provides a substantially clear, aqueous, sterile pharmaceutical composition useful as an anti-inflammatory agent comprising an active ingredient which is cromolyn, other bis-cromolyn derivatives or pharmaceutically active salts thereof, or mixtures thereof; greater than 20ppm of a transition metal or ion of Group IIA, IB, IIB or IVB of the Periodic Table; and a sufficient amount of a pharmaceutically acceptable chelating or sequestering agent to provide a clear solution.

Preferably the composition contains up to about 5% w/v of the active ingredient and at least about 0.15% w/v of sequestering agent. Preferably, also, the composition is tonicity-adjusted to an osmotic pressure in the range of about 200 mOsm to about 350 mOsm, has a pH in the range of about 4 to about 7, and contains a buffering agent.

The invention also includes a novel method for the formulation of the claimed composition.

According to the present invention, the sterile, clear, aqueous, tonicity-adjusted composition preferably comprises as an active ingredient a therapeutically useful amount of 1,3-bis(2-carboxychromon-5-yloxy)-propan-2-ol, or a pharmaceutically acceptable salt thereof, preferably the sodium salt. Other pharmaceutically acceptable salts include salts with potassium, lithium, ammonium salts and salts with organic bases, such as triethanolamine or diethylamine salts.

While sodium cromolyn is the preferred active ingredient, other bis-cromolyn compounds may be used which have been shown to exhibit anti-inflammatory action similar to, although slightly less effective than sodium cromolyn. Clear, stable pharmaceutical compositions of these compounds are within the scope of the present invention. Such compounds and their anti-inflammatory activity are disclosed in U.S. 3,777,033 which is incorporated herein by reference. These bis-cromolyn compounds have the formula

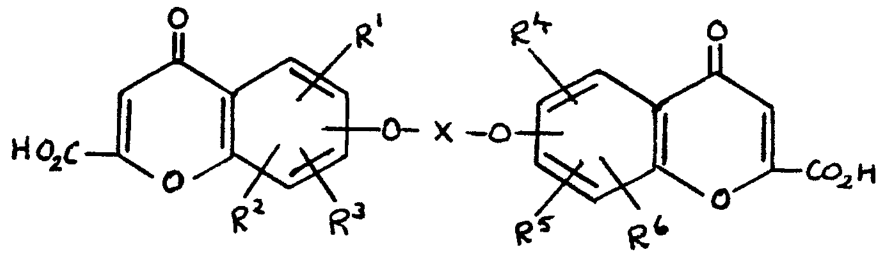

and including therapeutically acceptable salts, esters and amides thereof, in which $R^1$, $R^2$, $R^3$, $R^4$, $R^5$ and $R^6$ are each selected from the group consisting of hydrogen, halogen, hydroxy, lower alkyl, lower alkoxy, hydroxy-loweralkyl, halo-loweralkyl, hydroxyloweralkoxy, loweralkoxy-loweralkoxy and carboxyloweralkoxy; and X is selected from the group consisting of saturated and unsaturated, straight and branched hydrocarbon chains which may be interrupted by a member selected from the group consisting of benzene rings, dioxanyl, oxygen atoms and carbonyl groups, and which may be substituted by a member selected from the group consisting of halogen atoms, hydroxy groups and lower alkoxy groups.

Specific bis-cromolyn compounds are listed below in Table I. The protection as an anti-inflammatory agent is measured by the change in F.E.V.$_1$, and expressed as a percentage of protection as defined in 1U.S. 3,777,033. According to U.S. 3,777,033, the data in Table I is obtained by dissolving in sterile water at a concentration of 0.5%. Inhalation is administered for 5 minutes to a patient followed two hours later by challenge with antigen. The compounds and the percent of protection are listed below.

TABLE 1

| Compound under Test | Protection Percent |
|---|---|
| Disodium salt of 1,5-bis (2-carboxychromon-5-yloxy) pentane | 30-35 |
| Disodium salt of 1,7-bis (2-carboxychromon-5-yloxy-)-2,6 dihydroxy-4-oxaheptane | 25-30 |
| Disodium salt of 1,4-bis (2-carboxychromon-5-yloxy)butane | 45-50 |
| Disodium salt of 1,4-bis (2-carboxychromon-5-yloxy)-2,3 dihydroxy-butane | 40-45 |
| Disodium salt of 1,4-bis (2-carboxychromon-5-yloxy)-2 hydroxy-butane | 50-55 |
| Disodium salt of 1,4-bis (2-carboxychromon-5-yloxy)but-2-ene | 45-50 |
| Disodium salt of 1,10-bis (2-carboxychromon-5-yloxy)decane | 35-40 |
| Disodium salt of 1,6-bis (2-carboxychromon-5-yloxy)hexane | 45-50 |
| Disodium salt of 1,3-bis (2-carboxychromon-5-yloxy)-2-hydroxypropane | 65-70 |
| Disodium salt of 1,3-bis (2-carboxychromon-5-yloxy)propane | 40-45 |
| Disodium salt of 1,5-bis (2-carboxy 8-chlorochromon-5-xloxy)pentane | 20-25 |
| Disodium salt of 1,5-bis (2-carboxychromon-6-yloxy)pentane | 20-25 |
| Disodium salt of 1,5-bis (2-carboxychromon-7-yloxy)pentane | 45-50 |
| Disodium salt of 1,3-bis (2-carboxychromon-7-yloxy)-2-hydroxypropane | 45-45 |
| Disodium salt of 1,3-bis (2-carboxy 8-ethylchromon-5-yloxy)-2-hydroxypropane | 20-25 |
| Disodium salt of 1,5-bis (2-carboxychromon-5-yloxymethyl)benzene | 30-35 |
| Disodium salt of 1-(2-carboxychromon-5-yloxy)-3-2 carboxychromon-7-yloxy)-2-hydroxypropane | 40-45 |
| Disodium salt of 1,3-bis (2-carboxychromon-6-yloxy)-2-hydroxypropane | 15-20 |
| Disodium salt of 1,3-bis (2-carboxy-8-methylchromon-7-yloxy)-2-hydroxypropane | 25-30 |
| Disodium salt of 1,3-bis (2-carboxychromon-5-yloxy)-2-chloromethyl-2-hydroxymethylpropane | 40-45 |
| Disodium salt of 1,5-bis (2-carboxychromon-5-yloxy)-3-methylpentane | 20-25 |
| Disodium salt of 1 (2-carboxychromon-5-yloxy)-3-(2-carboxy-6-chloro-chromon-7-xyloxy)-2-hydroxypropane | 35-40 |
| Disodium salt of 1,3-bis (2-carboxychromon-5-yloxy)acetone | 30-35 |
| Disodium salt of 1,3-bis (2-carboxychromon-5-yloxy)-2-ethoxypropane | 30-35 |

The pharmaceutical solution can contain most preferably from about 0.8 to about 5% w/v of the cromolyn, preferably about 1% for the oral inhalation, about 4% for the nasal inhalation and from about 2 to 4% for ophthalmic use.

In addition to the active ingredient, the solution will also contain ionic species, such as, sodium chloride and potassium chloride, to adjust the tonicity. Preferably, the solution will contain about 0.05-1.0% w/v of potassium chloride, and sodium chloride, as needed. Other tonicity adjusting ingredients (to lower the sodium concentration) include glycerin, mannitol, and sorbitol.

Additionally, a preferred solution will contain about 0.016 to 0.02% w/v of calcium chloride dihydrate, which provides about 45 to 55 ppm of calcium ions in solution. The solution, however, can contain up to about 1100 ppm of calcium ions, and still be maintained as a clear, sterile, tonicity adjusted solution. The solution can contain greater than about 20 ppm of metal ions of transition metals or other metals of Group IIA, IB, IIB or IVB of the Periodic Table. For example, the solution can contain up to about 300 ppm of magnesium ions, up to about 5090 ppm of cupric ions, up to about 330 ppm of ferrous ions, up to about 2160 ppm of ferric ions, or up to about 7800 ppm of lead ions.

It has been surprisingly found that such high amounts of metal ions of transition metals or metals of Group IIA, IB, IIB or IVB can be present provided that there is up to about 5% w/v of a pharmaceutically acceptable chelating or sequestering agent also present in the solution. If there is about 20 ppm of such metal ions present, then the amount of chelating or sequestering agent present will be at least about 0.15% w/v. Sodium citrate dihydrate is the preferred sequestering agent, preferably present in an amount of 0.20 to 1.02% w/v, preferably 0.50%. Other sequestering or chelating agents can be utilized besides sodium citrate dihydrate, such as tartrates; sodium fumarate, maleate, or gluconate; phosphoric acid; ethylene diamine tetracetic acid or its salts and other di- or poly-anionic molecules. In general, the chelating or sequestering agent will be present in about 0.15-5% w/v for clear solutions containing up to about 5% w/v of cromolyn which have been adjusted for tonicity to an osmotic pressure in the range of about 200-350 mOsm.

Sodium acetate trihydrate in an amount of about 0.38 to 0.40% w/v, preferably 0.39% is also contained within the solution as a buffering agent. Typical buffering agents are disclosed in Lilker, E.S. Letter Brit. Med. J . (1982) 284 417, and include, but are not limited to, phosphate buffers, and the like.

A preferred physically stable aqueous, sterile, buffered, clear, tonicity adjusted formulation of cromolyn comprises cromolyn, or any of its pharmaceutically active salts, in a concentration of about 0.8 to 4.2% w/v, sodium chloride concentration adjusted to compensate for tonicity; 0.05 -0.10% potassium chloride; 0.016 - 0.02% calcium chloride dihydrate; 0.38 - 0.40% sodium acetate trihydrate; 0.20 -1.02% sodium citrate dihydrate, and sufficient mineral acid or base to adjust the pH to 4.0 - 7.0 with the remaining of the composition being purified water.

After dissolving all of the above ingredients in the solution, a sufficient amount of mineral acid such as hydrochloric acid, or base, preferably sodium hydroxide, can be added, if needed, to bring the pH to 4.0 to 7.0, with the remaining of the solution being purified water. Within the concentration ranges of the above ingredients, for an oral inhalation the solution will be isotonic or substantially isotonic, i.e., having an osmotic pressure of 300 mOsm ± 20 mOsm. The osmotic pressure can be adjusted as desired for an ophthalmic solution which may be hypotonic or for a nasal solution which will be isotonic, and generally formulations with an osmotic pressure in the range of about 200 to 350 mOsm will be useful.

Small amounts of pharmaceutically acceptable preservatives can also be added, such as, benzalkonium chloride, usually in an amount in the range of about 0.004-0.015% w/v.

It is surprising that the above solution can be formed which is clear and stable, in addition to being isotonic, in view of the teachings cf Patent No. 3,975,536 that solutions of cromolyn must contain less than about 20 ppm of metal ions (of groups IIA, IB, IIB, IVB or the transition metals) in the presence of a chelating agent.

The composition is made using conventional techniques, i.e., by adding all of the ingredients but the active ingredient to purified water, then adding the active ingredient to the resulting solution and stirring, filtering if necessary, then sterilizing the composition by autoclaving, for example, at a temperature of about 115°C for about 30 minutes. The autoclaving step can be omitted if the composition is produced by sterile filtration into a sterilized container under aseptic conditions.

The order of adding the ingredients to the solution is not particularly critical as long as the active ingredient is added after the chelating or sequestering and buffering agents have been added.

The preferred method for making the formulation is to add, in order, to purified water, sodium citrate, sodium acetate, calcium chloride, potassium chloride and sodium chloride in the desired amounts, then to mix the solution until all salts are dissolved. Then dilute hydrochloric acid or dilute NaOH is added to bring the pH most preferably to about 6.5-6.7. The sodium chromolyn is then added slowly, and mixing is

continued until all the ingredients are dissolved and the solution is clear. Then, the pH is adjusted again to 6.5.-6.7, if needed. The solution is then diluted with purified water to the desired concentration.

Alternatively, calcium chloride and sodium citrate are added to purified water, and the pH is adjusted. Then sodium acetate, potassium chloride, sodium chloride and sodium chromolyn are added in order, and the solution is stirred. The pH is adjusted, if necessary, then the solution is diluted to the desired concentration.

The composition, according to the present invention, can be used as a conventional aerosolized inhalation formulation or may be administered directly into the eye or nose. The dosage to be administered will vary with the condition to be treated, its severity and location; however, generally for use in the eye a dosage of about 1 to 2 drops (containing 1.6 to 3.2 mg of cromolyn sodium from a 4% solution) into the affected eye four to six times a day will be satisfactory. For nasal use the solution can be sprayed into each nostril 3 to 4 times daily (each spray dosage contains from 4 to 6 mg of cromolyn sodium). For use as an aerosolized inhalation a dosage of about 2 mL (about 20 mg of active ingredient) four times a day is useful.

The composition according to the present invention is useful as an ophthalmic preparation for the treatment of vernal kerato-conjunctivitis, vernal conjunctivitis, vernal keratitis, and giant papillary conjunctivitis. Nasal use is for patients with allergic rhinitis or hayfever. Bronchial asthmatics will utilize the composition by inhalation to reduce the intolerable side effects of sympathomimetic agents, reduce or eliminate the need of steroids or to abate asthma symptoms.

The compositions according to the present invention are also suitable for injection, i.e., intramuscularly, intravenously, or subcutaneously, since they are clear and substantially free of particulate matter.

The following examples are presented for the purpose of illustrating the invention and are not intended to constitute a limitation thereof.

## EXAMPLE 1

A sterile, clear, tonicity-adjusted formulation for oral inhalation is formed by mixing the following ingredients:

| | |
|---|---|
| Disodium Salt of 1,3-Bis (2-carboxychromon-5-yloxy)propan-2-ol | 1.0% w/v |
| Sodium Chloride | 0.5% w/v |
| Potassium Chloride | 0.075% w/v |
| Calcium Chloride Dihydrate (50 ppm calcium) | 0.018% w/v |
| Sodium Acetate Trihydrate | 0.39% w/v |
| Sodium Citrate Dihydrate | 0.50% w/v |
| HCl (0.5N)/NaOH (0.5N) | To pH 4.0-7.0 |
| Purified Water | To 100.0% |

## EXAMPLES 2-33

Sterile, clear, tonicity-adjusted formulations for oral inhalation were also formed by mixing the following ingredients:

| | % w/v NA | | | Na | | | Type ppm |
|---|---|---|---|---|---|---|---|
| metal Example | cromolyn | citrate | NaCl | KCl | NaOAc | ion | ion |
| 2 | 1 | 1 | 0.5 | .075 | 0.39 | $Mg^{+2}$ | 60 |
| 3 | 1 | 5 | 0.5 | .075 | 0.39 | $Mg^{+2}$ | 299 |
| 4 | 1 | 1 | 0.5 | .075 | 0.39 | $Ca^{+2}$ | 136 |
| 5 | 1 | 0.5 | 0.5 | .075 | 0.39 | $Ca^{+2}$ | 50 |
| 6 | 1 | 5 | 0.5 | .075 | 0.39 | $Ca^{+2}$ | 1090 |
| 7 | 1 | 5 | 0.5 | .075 | 0.39 | $Cu^{+2}$ | 250 |
| 8 | 1 | 5 | 0.5 | .075 | 0.39 | $Cu^{+2}$ | 509 |
| 9 | 1 | 5 | 0.5 | .075 | 0.39 | $Cu^{+2}$ | 2545 |
| 10 | 1 | 5 | 0.5 | .075 | 0.39 | $Cu^{+2}$ | 5091 |
| 11 | 1 | 5 | 0.5 | .075 | 0.39 | $Fe^{+2}$ | 332 |
| 12 | 1 | 5 | 0.5 | .075 | 0.39 | $Fe^{+3}$ | 270 |
| 13 | 1 | 5 | 0.5 | .075 | 0.39 | $Fe^{+3}$ | 539 |
| 14 | 1 | 5 | 0.5 | .075 | 0.39 | $Fe^{+3}$ | 1078 |
| 15 | 1 | 5 | 0.5 | .075 | 0.39 | $Fe^{+3}$ | 1617 |
| 16 | 1 | 5 | 0.5 | .075 | 0.39 | $Fe^{+3}$ | 2156 |
| 17 | 1 | 5 | 0.5 | .075 | 0.39 | $Pb^{+2}$ | 6256 |
| 18 | 1 | 5 | 0.5 | .075 | 0.39 | $Pb^{+2}$ | 7820 |
| 19 | 1 | 0.15 | 0.5 | .075 | 0.39 | $Ca^{+2}$ | 50 |
| 20 | 1 | 5.0 | 0.5 | .075 | 0.39 | $Ca^{+2}$ | 50 |
| 21 | 1 | 0.15* | 0.5 | .075 | 0.39 | $Ca^{+2}$ | 50 |
| 22 | 1 | 0.5* | 0.5 | .075 | 0.39 | $Ca^{+2}$ | 50 |
| 23 | 1 | 1.0* | 0.5 | .075 | 0.39 | $Ca^{+2}$ | 50 |
| 24 | 1 | 5.0* | 0.5 | .075 | 0.39 | $Ca^{+2}$ | 50 |
| 25 | 1 | 0.15** | 0.5 | .075 | 0.39 | $Ca^{+2}$ | 50 |
| 26 | 1 | 0.5** | 0.5 | .075 | 0.39 | $Ca^{+2}$ | 50 |
| 27 | 1 | 1.0** | 0.5 | .075 | 0.39 | $Ca^{+2}$ | 50 |
| 28 | 1[3] | 0.5 | 0.7 | - | - | $Ca^{+2}$ | 50 |
| 29 | 4[4] | 0.5 | 0.5 | .075 | 0.39 | $Ca^{+2}$ | 50 |
| 30 | 4[4] | 1.0 | 0.5 | .075 | 0.39 | $Ca^{+2}$ | 50 |
| 31 | 1 | 0.5 | 0.5 | .075 | 0.39 | $Ca^{+2}$ | 50 |
| 32 | 4 | 0.5 | 0.5 | .075 | 0.39 | $Ca^{+2}$ | 50 |
| 33 | 5 | 1.0 | 0.5 | .075 | 0.39 | $Ca^{+2}$ | 50 |

* Edetate disodium used instead of Na citrate.

** Phosphoric acid is used instead of Na citrate.

[3] Solution contains 0.16% potassium phosphate, monobasic, and 0.12% sodium phosphate, dibasic.

[4] Solution contains 0.005% benzalkonium chloride.

EXAMPLE 34

A sodium cromolyn (1,3-bis(2-Carboxychromon-5-yloxy)propan-2-ol solution was prepared by adding approximately 18 liters of USP Purified Water to a 25 liter tank equipped with a mixer.

The mixer was started and 125g of sodium citrate $2H_2O$ was added to the tank. This was followed by a period of mixing before the addition of 97.5g sodium acetate $3H_2O$. Mixing was continued until a clear solution was obtained.

Calcium chloride $2H_2O$ in the amount of 4.5g was then added with mixing until it was thoroughly dissolved. This was followed by the addition of 18.75g potassium chloride to the tank followed by thorough mixing. Next, 125g of sodium chloride were added to the tank followed by at least 15 minutes of mixing

until all the additions were dissolved and in solution as evidenced by visual inspection.

The pH was measured and adjusted with 1N HCl to provide a pH in the range of 6.5 to 6.7. If the pH dropped below this range, 1N NaOH was added to raise the pH to the desired range.

After the pH was adjusted and all of the other ingredients had been dissolved, 250g of sodium cromolyn (dried basis) were slowly added with mixing to the tank. Mixing was continued for about 10 hours when all of the sodium cromolyn was in solution. At the end of this period, the pH was checked and adjusted if needed with 1N HCl or 1N NaOH to a pH in the range of 6.5 to 6.7.

USP Purified water was then added to bring the total volume to 25 liters. A clear, stable, sterile solution was produced suitable for use as an anti-inflammatory agent.

EXAMPLE 35

Using the same proportions of ingredients as in Example 34 the following alternate mixing procedure is followed. Approximately 18 liters of USP Purified Water are added to a 25 liter tank equipped with a mixer.

The calcium chloride and sodium citrate are added to the purified water and thoroughly mixed until dissolved.

The pH is adjusted to 6.5 to 6.7 with 1N NaOH. Next the sodium acetate, potassium chloride, sodium chloride, and sodium cromolyn are added in that order. Each addition is followed by thorough mixing prior to the addition of the next ingredient.

When all of the ingredients are added and mixed to dissolution to produce a clear, solution, the pH is again adjusted to pH 6.5 to 6.7. Purified water is then added to bring the total volume to 25 liters.

The above examples demonstrate that the pharmaceutical composition of the invention is clear and stable even when greater than 20ppm of ions of transition metals or ions of Group IIA, IB, IIB or IVB of the Periodic Table are included. In fact, stable, clear solutions were obtained with as much as 5091ppm Cu ions; 7820ppm Pb ions; 2156 ppm Fe ions;and 299 ppm Mg ions. Thus, the composition of the invention provides an improvement over prior art compositions containing sodium chromolyn.

Various modifications of the inventions are contemplated and can be resorted to without departing from the spirit and scope of the invention as defined by the following appended claims.

**Claims**

1. A substantially clear, aqueous, sterile pharmaceutical composition useful as an anti-inflammatory agent comprising an active ingredient which is cromolyn, other bis-cromolyn derivatives or pharmaceutically active salts thereof, or mixtures thereof; greater than 20ppm of a transition metal or an ion of Group IIA, IB, IIB or IVB of the Periodic Table; and a sufficient amount of a pharmaceutically acceptable chelating or sequestering agent to provide a clear solution.

2. A composition as claimed in claim 1 wherein said bis-cromolyn derivative is a compound of the formula

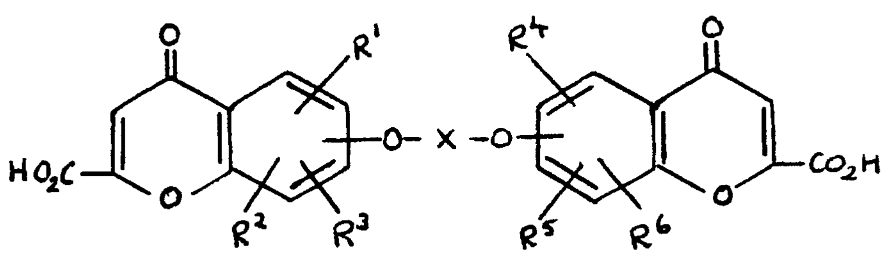

a therapeutically acceptable salt, ester or amide thereof, in which $R^1$, $R^2$, $R^3$, $R^4$, $R^5$ and $R^6$ are each selected from hydrogen, halogen, hydroxy, lower alkyl, lower alkoxy, hydroxyloweralkyl, haloloweralkyl, hydroxy-loweralkoxy, loweralkoxy-loweralkoxy or carboxy-loweralkoxy; and X is a saturated or unsaturated, straight or branched hydrocarbon chain which may be interrupted by a benzene ring, dioxanyl, oxygen atom or carbonyl group, and which may be substituted by a halogen atom, hydroxy group or a lower alkoxy group.

3. A composition as claimed in claim 1 or claim 2 wherein the bis-cromonyl derivative is in the form of a therapeutically acceptable salt.

4. A composition as claimed in any one of the preceding claims wherein the bis-cromlyn derivative is
disodium salt of 1,5-bis (2-carboxychromon-5-yloxy) pentane,
disodium salt of 1,7-bis (2-carboxychromon-5-yloxy)-2,6 dihydroxy-4-oxaheptane,
disodium salt of 1,4-bis (2-carboxychromon-5-yloxy)butane,
disodium salt of 1,4-bis (2-carboxychromon-5-yloxy)-2,3 dihydroxy-butane,
disodium salt of 1,4-bis (2-carboxychromon-5-yloxy)-2 hydroxy-butane,
disodium salt of 1,4-bis (2-carboxychromon-5-yloxy)but 2-ene,
disodium salt of 1,10-bis (2-carboxychromon-5-yloxy) decane,
disodium salt of 1,6-bis (2-carboxychromon-5-yloxy)hexane,
disodium salt of 1,3-bis (2-carboxychromon-5-yloxy)-2 hydroxypropane,
disodium salt of 1,3-bis (2-carboxychromon-5-yloxy)propane,
disodium salt of 1,5-bis (2-carboxy 8-chloro-chromon-5-xloxy)pentane,
disodium salt of 1,5-bis (2-carboxychromon-6-yloxy)pentane,
disodium salt of 1,5-bis (2-carboxychromon-7-yloxy)pentane,
disodium salt of 1,3-bis (2-carboxychromon-7-yloxy)-2-hydroxypropane,
disodium salt of 1,3-bis (2-carboxy 8-ethyl-chromon-5 yloxy)-2-hydroxypropane,
disodium salt of 1,5-bis (2-carboxychromon-5-yloxymethyl)benzene,
disodium salt of 1-(2-carboxychromon-5-yloxy)-3-2 carboxychromon-7-yloxy)-2-hydroxypropane,
disodium salt of 1,3-bis (2-carboxychromon-6-yloxy)-2-hydroxypropane,
disodium salt of 1,3-bis (2-carboxy-8-methylchromon-7-yloxy)-2-hydroxypropane,
disodium salt of 1,3-bis (2-carboxychromon-5-yloxy)-2-chloromethyl-2-hydroxymethylpropane,
disodium salt of 1,5-bis (2-carboxychromon-5-yloxy)-3-methylpentane,
disodium salt of 1 (2-carboxychromon-5-yloxy)-3-(2-carboxy-6-chloro-chromon-7-xyloxy)-2-hydroxypropane,
disodium salt of 1,3-bis (2-carboxychromon-5-yloxy)acetone,
disodium salt of 1,3-bis (2-carboxychromon-5-yloxy)-2-or ethoxypropane.

5. A composition as claimed in any one of the preceding claims wherein the active ingredient comprises the sodium salt of 1,3-bis(2-carboxychromon-5-yloxy)propan-2-ol.

6. A composition as claimed in any one of the preceding claims wherein the active ingredient is present in an amount of up to 5% w/v is present.

7. A composition as claimed in claim 6 wherein the active ingredient is present in an amount of up to 1% w/v.

8. A composition as claimed in any one of the preceding claims comprising at least 0.15% w/v of the chelating or sequestering agent.

9. A composition as claimed in claim 8 wherein the chelating or sequestering agent comprises sodium citrate.

10. A composition as claimed in any one of the preceding claims which contains about 1100 ppm of $Ca^{+2}$ ions, up to about 300 ppm of $Mg^{+2}$ ions, up to about 5090 ppm of $Cu^{+2}$ ions, up to about 330 ppm of $Fe^{+2}$ ions, up to about 2160 ppm of $Fe^{+3}$ ions, and/or up to about 7800 ppm of $Pb^{+2}$ ions.

11. A composition as claimed in any one of the preceding claims wherein the composition further comprises, if necessary, a sufficient amount of a pharmaceutically acceptable tonicity adjusting compound to provide an osmotic pressure in the range of from 200 mOsm to 350 mOsm.

12. A composition as claimed in claim 11 wherein the asmotic pressure is in the range of from 280-320 mOsm.

13. A composition as claimed in claim 11 or claim 12 wherein the composition is adjusted to a pH in the range of from 4 to 7.

14. A composition as claimed in any one of claims 11 to 13 wherein the tonicity adjusting compound is selected from sodium chloride, potassium chloride, calcium chloride, glycerin, mannitol or sorbitol.

15. A composition as claimed in any one of claims 11 to 14 which is tonicity-adjusted with a balanced salt solution which includes chlorides of sodium, potassium, calcium and/or magnesium.

16. A composition as claimed in any one of the preceding claims further comprising a pharmaceutically acceptable buffering agent or a preservative.

17. A substantially clear, sterile, aqueous, buffered, tonicity adjusted pharmaceutically composition comprising from 0.8 - 4.2% w/v 1,3-bis(2-carboxychromon-5-yloxy)propan-2-ol or a pharmaceutically acceptable salt thereof as an active ingredient; sodium chloride to adjust tonicity; 0.05 -0.1% potassium chloride; 0.016 - 0.020% calcium chloride dihydrate; 0.38 - 0.40% sodium acetate trihydrate; and 0.20 - 1.02% sodium citrate dihydrate at a pH of 4.0 to 7.0.

18. A composition as claimed in claim 17 wherein the active ingredient comprises the sodium salt of 1,3-bis(2-carboxychromon-5-yloxy)propan-2-ol.

19. A composition as claimed in claim 17 or claim 18 wherein said active ingredient is present in an amount of up to 1% w/v.

20. A method for making a substantially clear, sterile, aqueous pharmaceutical composition for use as an anti-inflammatory agent comprising an effective amount of an active ingredient which is cromolyn, other bis-cromolyn derivatives or pharmaceutically active salts thereof, or mixtures thereof; greater than 20ppm of a transition metal or an ion of Group IIA, IB, IIB or IVB of the Periodic Table; and a sufficient amount of a pharmaceutically acceptable chelating or sequestering agent to provide a clear solution which method comprises adding the bis-cromolyn compound to sterile water after adding the chelating or sequestering agent.

21. A method as claimed in claim 20 wherein the active ingredient is as defined in claim 2.

22. A method as claimed in claim 21 wherein the bis-cromolyn compound is 1,3-bis(2-carboxychromon-5-yloxy)propan-2-ol, or a pharmaceutically acceptable salt thereof.

Claims for the following Contracting States: ES, GR

1. A method for making a substantially clear, sterile, aqueous pharmaceutical composition for use as an antiflammatory agent, which method comprises admixing a pharmacologically effective amount of an active ingredient which is cromolyn, other bis-cromolyn derivatives or pharmaceutically acceptable salts thereof, or mixtures thereof and greater than 20ppm of a transition metal or an ion of Group IIA, IA, IIB or IVB of the Periodic Table with sterile water and a sufficient amount of a pharmaceutically acceptable chelating or sequestering agent to provide a clear solution.

2. A method as claimed in claim 1 wherein the active ingredient is a compound of the formula

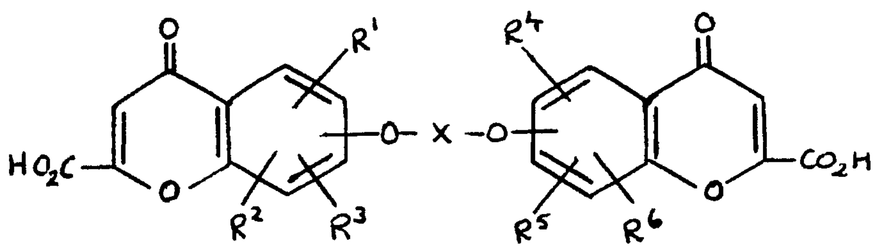

a therapeutically acceptable salt, ester or amide thereof, in which $R^1$, $R^2$, $R^3$, $R^4$, $R^5$ and $R^6$ are each selected from hydrogen, halogen, hydroxy, lower alkyl, lower alkoxy, hydroxyloweralkyl, haloloweralkyl, hydroxy-loweralkoxy, loweralkoxy-loweralkoxy or carboxy-loweralkoxy; and X is a saturated or unsaturated, straight or branched hydrocarbon chain which may be interrupted by a benzene ring, dioxanyl, oxygen atom or carbonyl group, and which may be substituted by a halogen atom, hydroxy group or a lower alkoxy group.

3. A method as claimed in claim 1 or claim 2 wherein the bis-cromonyl derivative is in the form of a therapeutically acceptable salt.

4. A method as claimed in any one of the preceding claims wherein the bis-cromlyn derivative is
disodium salt of 1,5-bis (2-carboxychromon-5-yloxy) pentane,
disodium salt of 1,7-bis (2-carboxychromon-5-yloxy)-2,6 dihydroxy-4-oxaheptane,
disodium salt of 1,4-bis (2-carboxychromon-5-yloxy)butane,
disodium salt of 1,4-bis (2-carboxychromon-5-yloxy)-2,3 dihydroxy-butane,
disodium salt of 1,4-bis (2-carboxychromon-5-yloxy)-2 hydroxy-butane,
disodium salt of 1,4-bis (2-carboxychromon-5-yloxy)but 2-ene,
disodium salt of 1,10-bis (2-carboxychromon-5-yloxy) decane,
disodium salt of 1,6-bis (2-carboxychromon-5-yloxy)hexane,
disodium salt of 1,3-bis (2-carboxychromon-5-yloxy)-2 hydroxypropane,
disodium salt of 1,3-bis (2-carboxychromon-5-yloxy)propane,
disodium salt of 1,5-bis (2-carboxy 8-chloro-chromon-5-xloxy)pentane,
disodium salt of 1,5-bis (2-carboxychromon-6-yloxy)pentane,
disodium salt of 1,5-bis (2-carboxychromon-7-yloxy)pentane,
disodium salt of 1,3-bis (2-carboxychromon-7-yloxy)-2-hydroxypropane,
disodium salt of 1,3-bis (2-carboxy 8-ethyl-chromon-5 yloxy)-2-hydroxypropane,
disodium salt of 1,5-bis (2-carboxychromon-5-yloxymethyl) benzene,
disodium salt of 1-(2-carboxychromon-5-yloxy)-3-2 carboxychromon-7-yloxy)-2-hydroxypropane,

disodium salt of 1,3-bis (2-carboxychromon-6-yloxy)-2-hydroxypropane,
disodium salt of 1,3-bis (2-carboxy-8-methylchromon-7-yloxy)-2-hydroxypropane,
disodium salt of 1,3-bis (2-carboxychromon-5-yloxy)-2-chloromethyl-2-hydroxymethylpropane,
disodium salt of 1,5-bis (2-carboxychromon-5-yloxy)-3-methylpentane,
disodium salt of 1 (2-carboxychromon-5-yloxy)-3-(2-carboxy-6-chloro-chromon-7-xyloxy)-2-hydroxypropane,
disodium salt of 1,3-bis (2-carboxychromon-5-yloxy)acetone,
or disodium salt of 1,3-bis (2-carboxychromon-5-yloxy)-2-ethoxypropane.

5. A method as claimed in any one of the preceding claims wherein the active ingredient comprises the sodium salt of 1,3-bis(2-carboxychromon-5-yloxy)propan-2-ol.

6. A method as claimed in any one of the preceding claims wherein the active ingredient is incorporated into the composition in an amount of up to 5% w/v is present.

7. A method as claimed in claim 6 wherein the active ingredient is incorporated into the composition in an amount of up to 1% w/v.

8. A method as claimed in any one of the preceding claims wherein the chelating or sequestering agent is incorporated into the composition in an amount of 0.15% w/v.

9. A method as claimed in claim 8 wherein the chelating or sequestering agent comprises sodium citrate.

10. A method as claimed in any one of the preceding claims wherein the bis-cromolyn compound is added to the sterile water after the addition of the chelating or sequestering agent.

11. A method as claimed in any one of the preceding claims wherein a tonicity adjusting compound is incorporated into the composition to provide an osmotic pressure in the range of from 200 mOsm to 350 mOsm.

12. A method as claimed in claim 11 wherein the tonicity is adjusted to from 280 mOsm to 320 mOsm.

13. A method as claimed in claim 11 or claim 12 wherein the tonicity adjusting compound is sodium chloride, potassium chloride, calcium chloride, glycerin, mannitol or sorbitol.

14. A method as claimed in any one of claims 11 to 13 wherein the tonicity adjustment is effected with a balanced salt solution which includes chlorides of sodium, potassium, calcium and/or magnesium.

15. A method as claimed in any one of the preceding claims wherein the pH of the composition is adjusted to a pH in the range of from 4 to 7.